(19) Europäisches Patentamt
European Patent Office
Office européen des brevets

(11) Publication number : **0 511 120 A1**

(12) # EUROPEAN PATENT APPLICATION

(21) Application number : **92401196.8**

(51) Int. Cl.$^5$ : **G01N 33/52**

(22) Date of filing : **24.04.92**

(30) Priority : **24.04.91 JP 122352/91**

(43) Date of publication of application :
**28.10.92 Bulletin 92/44**

(84) Designated Contracting States :
**BE DE FR GB IT NL SE**

(71) Applicant : **TERUMO Kabushiki Kaisha
44-1 Hatagaya 2-chome Shibuya-ku
Tokyo (JP)**

(72) Inventor : **Koike, Masufumi, c/o Terumo
Kabushiki Kaisha
1727-1, Tsuijiarai, Showa-cho
Nakakoma-gun, Yamanashi-ken (JP)**
Inventor : **Hayakawa, Tsuyoshi, c/o Terumo
Kabushiki Kaisha
1727-1, Tsuijiarai, Showa-cho
Nakakoma-gun, Yamanashi-ken (JP)**
Inventor : **Kanda, Zensho, c/o Terumo
Kabushiki Kaisha
1727-1, Tsuijiarai, Showa-cho
Nakakoma-gun, Yamanashi-ken (JP)**

(74) Representative : **Gillard, Marie-Louise et al
Cabinet Beau de Loménie 55, Rue
d'Amsterdam
F-75008 Paris (FR)**

(54) **Testing device.**

(57) A testing device (1), comprising a testing layer (2) provided with a reagent layer (4) capable of reacting with a specific component in a sample and consequently assuming a pertinent color, a retaining member (8) provided with an opening (7b) for supply of said sample and adapted to support said testing layer (2), and small air-passing holes (7c) formed in said retaining member (8) and opening into the ambient air.

FIG. 2

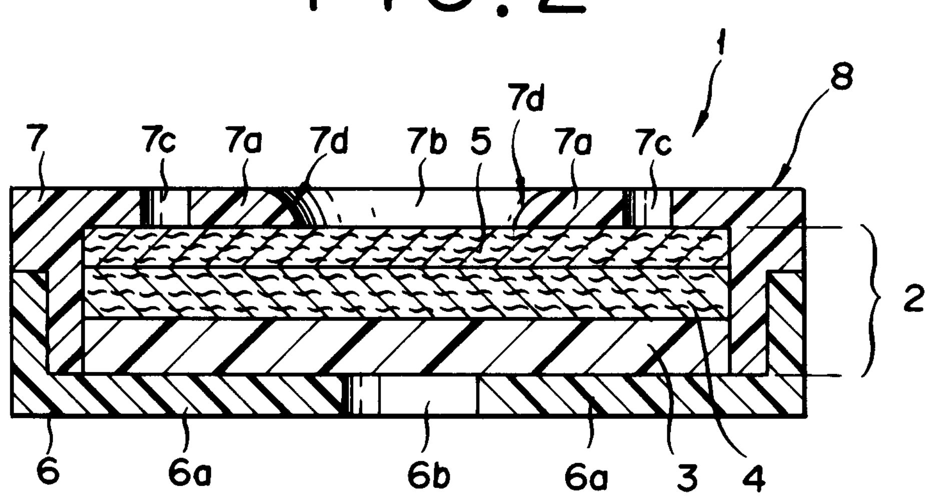




Jouve, 18, rue Saint-Denis, 75001 PARIS

## BACKGROUND OF THE INVENTION

### Field of the Invention:

This invention relates to a testing device for detecting and quantifying specific components in a sample under test.

### Description of the Prior Art:

Heretofore, in the detection of specific components in a given sample such as, for example, glucose, cholesterol, and uric acid in blood or glucose, hemoglobin, etc. in urine, the practice of using a testing paper which comprises a sheetlike substrate formed of resin such as, for example, polyethylene terephthalate and a reagent layer superposed on the substrate and having deposited therein reagents capable of reacting with the specific components and consequently assuming pertinent colors has been in vogue. Since the intensities of coloration (color densities) occurring in the reagent layer of the testing device are proportionate to the amounts of specific components in the sample, these specific components in the sample can be quantified by optically determining the intensities of coloration in the reagent layer. A modified version of the testing paper which is additionally provided on the reagent layer thereof with a developing layer serving to develop uniformly a given sample has been proposed to the art.

For the purpose of imparting to the testing paper of this nature improved operability and improved permeability to a sample, a testing device having a testing paper nipped between opposed retaining members has been disclosed (JP-A-2-179,450(1990)). The retaining members of this testing device are provided with an opening for supplying a given sample to the testing paper and an opening for allowing determination of intensities of coloration occurring in the reagent layer. In addition, a testing device which has a waterproofing layer superposed on the developing layer and has at least one small hole for supply of a sample formed in the waterproofing layer has been disclosed (US-A-4,631,174). This testing device is enabled by the waterproofing layer thereof to prevent the moisture in the reagent layer from evaporation and consequently impart improved conserving property to the reagent layer.

The testing device of this construction is shielded from the ambient air except for openings or small holes formed in one component layer or other thereof and, therefore, entails the disadvantage that it prevents the reagent layer from being uniformly supplied throughout the entire surface thereof with a gas (oxygen in the air) necessary for coloring reactions and suffers the reagent layer to produce uneven coloration. Particularly, the testing device taught by US-A-4,631,174 intends the waterproofing layer to prevent the moisture from evaporation and compels the sample to be supplied in such a manner as to cover the small hole. As a result, the small hole is filled to capacity with the sample and the reagent layer is shut off from the ambient air and is no longer supplied with oxygen so that the reaction of coloration proceeds incompletely or the time for the test elongates.

An object of this invention, therefore, is to provide a novel testing device.

Another object of this invention is to provide a testing device which precludes uneven coloration and allows the required determination to be performed quickly and accurately.

## SUMMARY OF THE INVENTION

These objects are accomplished by a testing device which comprises a testing layer provided with a reagent layer capable of reacting with specific components in a sample and consequently assuming pertinent colors, a retaining member provided with an opening for supply of the sample and adapted to retain the testing layer, and small gas holes formed in the retaining member and opened into the ambient air.

This invention provides a testing device in which the retaining member is composed of a pair of mutually fitting retaining pieces and the testing layer is nipped between the retaining pieces. This invention also provides a testing device in which the small gas holes are formed as disposed round the periphery of the opening. This invention further provides a testing device in which the opening is formed in one of the two retaining pieces and the small gas hole is formed in the other retaining piece. This invention provides a testing device in which the small gas holes have a diameter in the range of from 0.5 to 5 mm. This invention further provides a testing device in which the testing layer is pervious to light. The testing device according to the invention may advantageously include means for optical determination.

In the testing device of this invention, since the small holes for passage of air are formed in the retaining member and the oxygen necessary for the reactions of coloration is uniformly supplied through these small holes to the reagent layer, the reactions for coloration are promoted and the uneven coloration is prevented. Thus, the result of accurate detection can be obtained with a short duration of determination. Even when the

sample is inadvertently dropped in an unduly large amount, therefore, the reduction in the duration of determination aimed at by this invention is ensured because the sample is absorbed additionally through the small holes.

BRIEF DESCRIPTION OF THE DRAWINGS

Fig. 1 is a plan view illustrating a testing device as one embodiment of this invention,
Fig. 2 is an enlarged cross section taken through Fig. 1 along the line II-II,
Fig. 3 is an enlarged cross section illustrating a testing device as another embodiment of this invention similarly to Fig. 2, and
Fig. 4 is an enlarged cross section illustrating a testing device as yet another embodiment of this invention similarly to Fig. 2.

DESCRIPTION OF THE PREFERRED EMBODIMENTS

Now, this invention will be described more specifically below with reference to preferred embodiments illustrated in the accompanying drawings.

Fig. 1 is a plan view illustrating one embodiment of the construction of a testing device of this invention and Fig. 2 is a cross section taken through the testing device of Fig. 1 across the line II-II. In the drawing of Fig. 2, the component layers are depicted each in an exaggerated thickness. As illustrated in these drawings, a testing device 1 has a testing layer 2 carried on a retaining member which will be more specifically described herein below. The testing layer 2 has a supporting member 3, a reagent layer 4, and a developing layer 5 sequentially superposed in the order mentioned. The supporting member 3 has the shape of a plate of a thickness approximately in the range of from 20 to 700 μm, preferably from 50 to 300 μm, and size approximately 8 to 20 mm X 8 to 20 mm, and is made of a material inactive to the sample. The supporting member may be provided with a grip portion 3a, if necessary. In such case, the size is 8 to 20 mm X 40 to 70 mm, and a projection 3b may be formed on the head surface or the tail surface of the grip portion 3a, so that the head or the tail of the testing device is easily distinguished.

The materials which are effectively usable for the supporting member 3 include various resins such as polyethylene terephthalate, cellulose esters (including cellulose diacetate, cellulose acetate, cellulose acetate propionate, etc.), bisphenol A type polycarbonate, polymethyl methacrylate, polyvinyl chloride, polypropylene, polystyrene, and polyvinyl alcohol, and glass, for example. Optionally, the supporting member 3 may be what is obtained by superposing sheets of two or more different materials. This supporting member 3 is preferable to be pervious to light, namely to be transparent or translucent.

The reagent layer 4 has deposited on a carrier a reagent which will be more specifically described herein below. This carrier is desired to be formed of a nonfibrous or fibrous porous material.

The nonfibrous porous materials which are effectively usable for the carrier include dispersions having such porous substances as diatomaceous earth and microcrystalline substances dispersed in a binding agent and porous aggregates having minute spherical beads of glass and resin joined point to point as well as a membrane filter which is representative of nonfibrous porous materials.

As fibrous porous materials, woven fabrics, knitted fabrics, non-woven fabrics, filter papers, and aggregates of short fibers may be cited. Here, the woven fabrics and knitted fabrics embrace woven fabrics, knitted fabrics, and articles similar thereto. The textures of woven fabrics include those of all kinds which are currently in actual use. Examples are plain weave, twill weave, and satin weaves.

The texture of knitted fabrics is not particularly restricted but may be freely selected from among weft knitting (jersey), warp knitting (tricot), circular knit, plain knit, and stockinette, for example.

The carrier which is formed of woven fabric or knitted fabric as described above excels the reagent layer which is formed by applying to the carrier a coating liquid which will be more specifically described herein below and drying the applied layer of the coating liquid in terms of permeability to the sample and the components thereof and perviousness to such a gas as oxygen which is necessary for the reactions of coloration. Particularly, since the carrier permits easy permeation therein of such oily components as cholesterol, it can be ideally used in a testing device intended for detection of such components.

When the carrier for the reagent layer 4 is formed in a texture of plain weave or plain knit which has regularity of pattern, it proves desirable in respect that it permits uniform permeation of a given sample therethrough and allows preclusion of uneven coloration by uniformizing the amount of the sample to be distributed per unit area in the reagent layer 4. As examples of the fiber which is effectively usable for the construction of a fibrous porous material represented by a woven or knitted fabric, natural fibers such as cotton, kapok, flax, hemp, ramie, silk, and wool, chemical fibers such as nylon, polyester, rayon, cuprammonium rayon, acetate, vinylon, acryl, and

polyethylene terephthalate, and combinations of two or more fibers selected from among the natural and chemical fibers enumerated above (such as, for example, mixted fibers of nylon and polyester) may be cited. The thickness of these fibers, though variable with the particular kind of fibers, generally is approximately in the range of from 0.1 to 1.0 denier. The carrier of this construction is desired to exhibit hydrophilicity. The reason for the hydrophilicity is that it aids in accelerating permeation and diffusion of the sample in the carrier. This hydrophilicity may either originate in the material itself which forms the carrier (as when the carrier is formed of fibers of cotton, silk, or nylon) or arise from the treatment given to the carrier for impartation of hydrophilicity thereto.

The impartation of hydrophilicity may be attained by a simple method which solely comprises thoroughly washing a given carrier thereby denuding the carrier of a sizing agent and other processing agents. Preferably, it is attained by incorporating in the web of the carrier a surfactant which will be more specifically described herein below. The incorporation of the surfactant is accomplished, for example, by a method which comprises either immersing the washed carrier in a solution of the surfactant (0.05 to 5%) or spraying the solution of the surfactant onto the carrier and subsequently drying the wet carrier.

The surfactant may be any of water-soluble nonionic, cationic, anionic, and amphoteric surfactants. Particularly, nonionic surfactants such as, for example, dimethyl siloxane-methyl(polyoxyethylene) copolymers, alkylaryl ethers such as polyoxyethylene and polyglycerol, aliphatic esters, and sorbitan esters prove preferable. Besides, sodium di-2-ethylhexyl sulfosuccinate (Aerosol-OT), Triton X-100, sodium laurylsulfate, and sodium cholate are usable.

The impartation of hydrophilicity to the carrier can be otherwise attained by a method which comprises wetting a woven or knitted fabric with either an aqueous solution of such a hydrophilic polymer as polyvinyl alcohol or an aqueous hydrophilic polymer solution containing a fine powder of titanium dioxide or barium sulfate or a wetting agent such as glycerol or polyethylene glycol and subsequently drying the wet woven or knitted fabric. In this case, the concentration of the hydrophilic polymer in the aqueous solution thereof is desired to be approximately in the range of from 0.1 to 5.0% by weight. It is also permissible to obtain the impartation of hydrophilicity to the carrier by subjecting the carrier as to a plasma treatment. The thickness of the reagent layer 4, though variable with the material and behavior of the carrier, generally is desired to be approximately in the range of from 1 to 500 μm, preferably from 5 to 300 μm.

The reagent layer 4 has deposited therein a reagent which will be more specifically described herein below. The composition of this reagent is properly decided, depending on the particular components to be detected (quantified) in a given sample. In the detection of grape sugar in a sample, for example, glucose oxidase (GOX) and peroxidase (POD) which are enzymes and a coloring agent (color principle) constitute themselves main components for the reagent.

In the place of the enzymes mentioned above, cholesterol oxidase, cholesterol esterase and peroxidase, riboprotein lipase and glycerol oxidase and peroxidase, and phsopholipase D and choline oxidase and peroxidase may be used.

In the detection of occult blood (hemoglobin) in a sample (urine), a hydroperoxide and a coloring agent constitute themselves main reagents for the reagent. Specifically, hydroperoxides include bis [4-(alphahydroperoxyisopropyl)benzyl] ether, 2,5-dimethylhexane-2,5-dihydroperoxide, cumene hydroperoxide, 2,5-dimethylhexanone-2,5-dihydroperoxide, diisopropyl benzene hydroperoxide, t-butyl hydroxy peroxide, p-menthane hydroperoxide, and 4-methylphenyl isopropyl hydroperoxide, for example.

The coloring agents which are effectively usable herein include benzidines such as o-tolidine, m-tolidine, benzidine, tetramethyl benzidine, o-methyl benzidine, and o-dianisidine, and derivatives thereof, 2,7-diaminofluorene, 4-aminoantipyrin (4-AAP), and combinations of 4-APP with a coupling agent capable of forming a coupling thereof, for example.

The coupling agents which are effectively usable herein include phenol derivatives such as p-chlorophenol, 2,4-dichlorophenol, 2,4-dibromophenol, and 2,4,6-trichlorophenol, naphthalene derivatives such as 4-chloro-1-naphthalene and 1,7-dihydronaphthalene, and aniline derivatives such as N,N-dimethylaniline, N,N-diethyl-m-toluidine, N-ethyl-N-sulfopropyl-m-toluidine, N-ethyl-N-(2-hydroxy-3-sulfopropyl-m-toluidine (TOOS), 5,6,7,8-tetrahydro-1-naphthylamine, N-ethyl-N-(2-hydroxy-3-sulfopropyl)-3,5-dimethoxy aniline, for example. The other coloring agents which are effectively usable herein include 4,4′-diaminodiphenyl, various substituted phenylene diamines such as o-phenylene diamine, m-phenylene diamine, p-phenylene diamine, 2,3-tolylenediamine, 2,4-tolylenediamine, 2,5-tolylenediamine, and 2,6-tolylenediamine, pyrogallic acid, gallic acid, phenols such as fluoroglucinol, hydroquinone, and leucoindophenol, guaiacol, pyridine derivatives, substituted azines, and leucomalachite green, for example.

The deposition of such a reagent in the carrier is carried out, for example, by immersing the carrier in a solution containing the reagent or spraying the same solution onto the carrier and subsequently drying the wet carrier. The reagent layer 4, when necessary, may incorporate therein additives such as, for example, pH adjusting agent, light-reflecting substance, stabilizer, sensitizer, oxidizing agent, wetting agent, and viscosity enhancer in addition to the aforementioned surfactant.

The pH adjusting agents which are effectively usable herein include phosphoric acid buffer solution, citric acid buffer solution, boric acid buffer solution, tris buffer solution, and good buffer solution such as N-2-hydroxyethyl piperazine-N-2-ethanesulfonic acid (HEPES) buffer solution, for example. The light reflecting substance is intended, when whole blood is a sample, to exclude the possible effect of red blood corpuscles on the determination of color density. The light reflecting substances which are effectively usable herein include titanium dioxide, barium sulfate, aluminum, and various ceramic substances which are offered in the form of fine powder. The stabilizers which are usable for the purpose of enhancing the stability of produced color include the copolymer of methylvinyl ether and maleic anhydride and half alkyl esters thereof, for example.

As stabilizers usable for the purpose of preventing the testing device in storage from deterioration by aging, aryl semicarbazoles such as, for example, 1-phenyl semicarbazole and mercaptoimidazoles such as, for example, 2-mercaptobenzimidazole and 2-mercaptomethylbenzimidazole which are disclosed in US-A-4,220,713 and JP-A-3-103,194(1991) may be cited.

The sensitizer is intended to enhance the peroxidase activity, for example, in hemoglobin. The sensitizers which are effectively usable herein include quinoline and derivatives thereof such as, for example, quinine, cinchonine, 6-methoxyquinoline, quinaldine, 8-amino-6-methoxyquinoline, 2-quinolinol, isoquinoline, benzo(f)quinoline, and 3-aminoquinoline.

The oxidizing agent is intended to exclude the possible effect of such a reducing substance as ascorbic acid which is present in a sample. Preferably, oxygen acids, salts and metal salts thereof are used. As oxygen acids and salts thereof, $MXO$, $MXO_3$, $MXO_4$, $M_3H_2XO_6$ (wherein M is atoms such as, for example, Na, K, and H which are capable of forming monovalent cations and X is halogen atoms such as, for example, I, Br, and Cl). Specifically, they are $HClO$, $NaClO$, $KClO$, $HBrO$, $NaBrO$, $KBrO$, $HIO$, $NaIO$, $KIO$, $HClO_3$, $NaClO_3$, $KClO_3$, $HBrO_3$, $NaBrO_3$, $KBrO_3$, $NaIO_3$, $KIO_3$, $HClO_4$, $NaClO_4$, $KClO_4$, $HBrO_4$, $NaBrO_4$, $KBrO_4$, $HIO_4$, $NaIO_4$, $KIO_4$, $Na_3H_2IO_6$, and $K_3H_2IO_6$, for example. The metal salts of oxygen acids include ferric chloride, cupric chloride, copper sulfate, copper acetate, mercury acetate, bismuth acetate, and lead acetate, etc.

The wetting agents which are effectively usable herein include alkyl sulfates such as polyvinyl pyrrolidone, sodium laurylsulfate, sodium dodecylsulfate, and sodium tetradecylsulfate, alkyl benzenesulfonates such as sodium dodecylbenzenesulfonate, and dialkyl sulfosuccinates such as sodium di-2-ethylsulfosuccinate and sodium diheptylsulfosuccinate, for example.

The viscosity enhancers which are effectively usable herein include such polymers as polyvinyl alcohol, polyvinyl pyrrolidone, polyethylene glycol, polyacrylicacid salts, polyacryl amide, poly(hydroxyethyl methacrylate), poly(hydroxyethyl acrylate), and carboxymethyl cellulose, gelatine, and gum arabic, for example.

The reagent layer 4 needs not be limited to what relies on the aforementioned carrier but may be what is formed by causing a coating liquid containing a binding agent represented by gelatin and the aforementioned reagent and additives to adhere to the surface of the supporting member 3 by application, immersion, or spraying and subsequently drying the applied layer of the coating liquid.

The other binding agents than gelatin include polyvinyl alcohol, polyvinyl pyrrolidone, agarose, sodium polyvinylbenzene sulfonate, and polyvinyl propionate, for example.

The reagent layer may incorporate therein a film-hardening agent which is capable of cross-linking the binding agent such as gelatin. The film-hardening agents which are effectively usable herein include aziridine derivatives such as N,N′-hexamethylene-1,6-bis(1-aziridine carboxamide) (HDU), trimethylol propane-tri-β-aziridinyl propionate (TAZM), and tetramethylol methane-tri-β-aziridinyl propionate (TAZO) and compounds of the epoxy type, the acryloyl type, the vinylsulfonyl type, and the chlorotriazine type, for example.

The testing device may use two or more such reagent layers as described above. In this case, the component reagent layers may have severally different reagents deposited therein so that the testing device will be capable of detecting two or more specific components present in a given sample.

The developing layer 5 functions to effect substantially uniform development of a given sample on the reagent layer 4. This developing layer 5 is formed of a fibrous or nonfibrous porous material such as, for example, mesh, woven fabric, knitted fabric, non-woven fabric, filter paper, or membrane filter. Among other materials mentioned above, the mesh (or a net) proves particularly preferable. The mesh is excellent in the ability to develop or spread a sample, particularly capable of uniformly developing quickly even a sample such as blood or saliva which has relatively high viscosity, and pervious to gases. Depending on the largeness of openings in the mesh, this mesh is capable of retaining this perviousness to gases even when it is wetted with a sample and allowing the supply of a gas to the reagent layer 4 which will be more specifically described herein below.

The term "mesh" as used in this invention refers to a sheetlike member possessing regularly arranged openings. Morphologically, this mesh is a woven or knitted sheet of fibers, an one-piece molded sheet, or a fabricated sheet. The materials which are effectively usable for constructing this mesh include polyamide, rayon, polypropylene, polyethylene, polyvinyl chloride, polyesters such as polyethylene terephthalate and polybutylene terephthalate, and polyolefins, for example. The fibers forming the mesh are preferable to have diameters approximately in the range of from 50 to 200 μm, preferably from 60 to 150 μm.

The size of openings of the mesh is preferable to be approximately in the range of 20 to 300 threads, preferably from 40 to 100 threads, per inch. The reason for this particular range is that the ability of the mesh to develop a sample is poor if the size is less than 20 threads per inch, whereas the perviousness of the mesh to gases particularly in a wet state is inferior if the size exceeds 300 threads per inch,(1 inch = 2.54 cm).

The developing layer 5 represented by such a mesh as described above is preferable to have been given a treatment for impartation of hydrophilicity and consequent enhancement of the ability to develop a sample. This treatment consists in thorough washing of the web of the developing layer 5 or incorporation of a surfactant in the web to be performed in the same manner as described above.

The testing layer 2 constructed as described above, as illustrated in Fig. 2, is nipped by a retaining member 8 and adapted to fix the developing layer 5, the reagent layer 4, and the supporting member collectively in a pressed state.

The retaining member 8 is composed of a first retaining piece 6 and a second retaining piece 7 which are adapted to fit each other and, in their mutually fit state, exert prescribed pressure on the peripheral part of the testing layer 2. In the substantially central portion of the first and second retaining parts 6 and 7 (on the insides of nipping parts 6a and 7a), circular openings 6b and 7b are same axially formed respectively. The circular opening 7b is preferably formed in the larger diameter than that of the circular opening 6b for the more adequate detection of the uniform development portion. The diameter of the circular opening 6b is preferably about 60-90% of the diameter of the circular opening 7b.

The opening 6b of the first retaining piece 6 is intended to serve as a window for allowing an optical measuring instrument (analyzer) (not shown) on the supporting member 2 side to project light and receive reflected light and determine the intensities of colors produced in the reagent layer 4. In contrast thereto, the opening 7b of the second retaining piece 7 is intended as an open space for allowing supply of a sample to the testing layer 2. The diameter of the opening 6b is preferable to be approximately in the range of from 1 to 20 mm, preferably 4 to 10 mm. The diameter of the opening 7b is preferable to be approximately in the range of from 1 to 20 mm, preferably from 3 to 10 mm. The edge portion 7d of the circular opening 7b is preferably chamfered and inclined at about 40-60° for easily dropping the specimen. The shape of these openings 6b and 7b needs not be limited to a circle as illustrated but may be an ellipse or a regular polygon, for example.

The material for the first and second retaining pieces 6 and 7 has no particular restriction except for the sole requirement that it should be impervious to liquid. The materials which are effectively usable herein include various resins represented by polyethylene, polypropylene, polyvinyl chloride, polystyrene, polyamide, polycarbonate, polyesters such as polyethylene terephthalate and polybutylene terephthalate, ABS resin, and acrylic resins such as polymethyl methacrylate, ceramics represented by alumina and silica, and metals represented by aluminum, stainless steel, and titanium, for example.

In the retaining member 8 constructed as described above, a plurality of small air-passing holes 7c piercing the nipping part 7a are disposed round the opening 7b in the nipping part 7a of the second retaining piece 7. Owing to the provision of these small holes 7c, the ambient air is supplied even to the portions of the reagent layer 4 underlying the nipping parts 6a and 7a and, as a result, substantially uniform supply of oxygen necessary for the reactions of coloration throughout the entire area of the reagent layer 4 is ensured and the possible uneven coloration is prevented.

These small holes 7c, therefore, are preferable to be formed as uniformly distributed round the opening 7b. The number of these small holes is desired to be approximately in the range of from 1 to 100, preferably from 4 to 10.

The diameter of the small holes 7c is preferable to be approximately in the range of from 0.5 to 5 mm, preferably 0.7 to 3 mm. The reason for this particular range is that the ability of the holes to allow passage of air is not sufficient, though depending on the number of small holes to be formed, if the diameter is less than 0.5 mm, whereas the retention of the testing layer 2 is not sufficient if the diameter exceeds 5 mm. The shape of the small holes 7c needs not be limited to a circle as illustrated but may be selected from among ellipse, polygons such as triangle, square, hexagon, and octagon, a slit, and a net, for example.

Though Fig. 1 and Fig. 2 depict the first retaining piece 6 and the second retaining piece 7 as separately formed components, this invention allows these two retaining pieces to be connected to each other or to be integrally formed. These two retaining pieces may be joined to each other across one side each thereof and the part joining them may be endowed with flexibility so that the two joined retaining pieces will be rotated around the joining part.

Now, the operation of the testing device 1 will be described below. When a sample of an amount approximately in the range of from 3 to 100 μl is dropped into the opening 7b of the testing device 1 by the use of a

pipet, for example, this sample is uniformly developed on the developing layer 5 and then allowed to permeate the reagent layer 4. After the elapse of a prescribed time, a specific component of the sample to be detected reactss ith the reagent in the reagent layer 4 and assumes a relevant color. When the testing layer 2 is intended to detect grape sugar in the sample, for example, a reaction represented by the following formula 1 takes place. When the testing layer 2 is intended to detect cholesterol in the sample, a reaction represented by the following formula 2 occurs.

$$\text{Glucose} + O_2 \xrightarrow{\text{GOX}} \text{gluconic acid} + H_2O_2$$
$$H_2O_2 \xrightarrow{\text{POD}} [O] + H_2O$$
$$[O] + \text{color principle} \longrightarrow \text{pigment}$$

$$\text{Cholesterol ester} \xrightarrow{\text{COE}} \text{cholesterol} + \text{free fatty acid}$$
$$\text{Cholesterol} + O_2 \xrightarrow{\text{COD}} \Delta^4\text{-cholesterol} + H_2O_2$$
$$H_2O_2 \xrightarrow{\text{POD}} [O] + H_2O$$
$$[O] + \text{color principle} \longrightarrow \text{pigment}$$

In such case, oxygen in the formulas 1 and 2 is supplied from atmosphere. That is, oxygen in air is introduced from a small hole 7c and is supplied to the reagent layer 4 through the developing layer 5. The above mentioned reaction generates through the whole area of the reagent layer 4 by such supply and coloration unevenness can be prevented.

The intensity of coloration of the colored part thus obtained is determined by visual observation or with the aid of an optical instrument. In the case of the optical determination, the optical instrument (analyzer) emits a light which is projected via an opening 6b from the first retaining piece 6 side and then receives the light to be returned by reflection from the testing layer and consequently determines the absorbence of the reflected light. The testing device la of this invention contributes to shortening the time required for the determination because the sample suffered to overflow the opening 7b when the sample is inadvertently supplied in an unduly large amount (50 to 100 μl or more, for example) to the opening 7b is absorbed additionally through the small holes 7C.

Fig. 3 is a cross section illustrating a testing device as another embodiment of this invention. In this drawing, the component layers are depicted each in an exaggerated thickness. The points in which the testing device 11 illustrated in Fig. 3 differs from the testing device 1 mentioned above will be described below. The points shared by these two devices will be omitted from the following description.

A retaining member 18 of a testing device 11 has a circular protuberance 17d formed along the periphery of an opening 17b of a second retaining piece 17 as projected toward the inside of the retaining member 18. The leading end of this protuberance 17d collides against a developing layer 15 and exerts pressure thereon to fix the component layers of a testing layer 12. Owing to the provision of the protuberance 17d in the manner described on the rear side of the second retaining piece 17, a supporting member 13, a reagent layer 14, and the developing layer are infallibly prevented from mutual deviation and separation and are allowed to be joined with uniform pressure. As a result, the otherwise possible uneven coloration is prevented and the accuracy of determination can be enhanced. The edge portion 17e of the opening 17b is preferably chamfered as preceding embodiment. The testing layer 12 of the testing device 11 is provided with a sample-absorbing layer 19 adapted to absorb any excess sample. To be specific, the sample-absorbing layer 19 is disposed in the part of the second retaining piece 17 which is enclosed with a nipping part 17a, a protuberance 17d, and the developing layer 15.

The sample-absorbing layer 19 is required to be pervious to gases. The materials which are effectively usable herein include fibrous porous materials such as woven fabric, knitted fabric, non-woven fabric, and filter paper and nonfibrous porous materials such as foamed polyvinyl alcohol, for example.

In the testing device constructed as described above, the ambient air is introduced via small holes 17c, passed sequentially through the sample-absorbing layer 19 and the developing layer 15 in the order mentioned, and supplied to the reagent layer 14. In Fig. 3, the reference numerals which are sums of reference numerals of Fig. 2 plus 10 represent the same members as illustrated in Fig. 2.

Fig. 4 is a cross section illustrating a testing device as yet another embodiment of this invention. In this drawing, the component layers are depicted each in an exaggerated thickness. The points in which a testing

device illustrated in Fig. 4 differs from the testing device 1 and the testing device 11 mentioned above will be described below. The points shared by these three testing devices will be omitted from the following description.

A testing layer 22 in the testing device 21 is not provided with any countertype of the aforementioned supporting member 13 but is composed, similarly to the embodiments cited above, of a reagent layer 24, a developing layer 25, and a sample-absorbing layer 29.

A retaining member 28 of the testing device 21 is provided in the periphery of an opening 27b of a second retaining piece 27 thereof with a protuberance 27d similar to the countertype of the preceding embodiment. The edge portion 27e of the opening 27b is preferably chamfered as preceding embodiment. A nipping part 27a of the second retaining piece 27 has formed therein no countertype of the small holes 17c.

A first retaining piece 26 has formed therein no countertype of the opening 16b for optical determination. To be specific, the first retaining piece 26 functions as a supporting member and possesses in itself perviousness to light and allows the projected light and the reflected light to be passed through a central part 26c thereof and consequently permits determination of the intensity of color produced in the reagent layer 24. Small holes 26c for passage of air are formed as disposed around the central part 26d of the first retaining piece 26. The small holes 26c are identical to the small holes 27c mentioned above. In the testing device 21 constructed as described above, the ambient air is introduced through the small holes 26c and supplied to the reagent layer 24. In Fig. 4, the reference numerals which are sums of reference numerals of Fig. 2 plus 20 represent the same components as illustrated in Fig. 2.

The testing device of this invention can be used for the detection of glucose, uric acid, BUN, creatinine, calcium, oxalic acid, proteins, lipoproteins (LDL, HDL), cholesterol, triglyceride (neutral fat), free fatty acids, ketones, bilirubin, urobilinogen, nitrites, ascorbic acid, hemoglobin, leukocyte, GOT, GPT, ALP, and gamma-GT in various samples (such as, for example, whole blood, blood plasma, blood serum, urea, excrement, saliva, lymphatic fluid, cerebrospinal fluid, and other body fluids). Optionally, the testing device may be designed as a multi-item testing device capable of detecting two or more components mentioned above. It also finds utility in other fields such as the analysis of foodstuffs and environmental specimens, for example.

Now, this invention will be described more specifically below with reference to a working example.

A testing device of the construction illustrated in Fig. 3 was produced as follows.

1. Supporting member:

A piece, 20 mm (width) x 45 mm (length) in size, cut from a polyethylene terephthalate film 200 μm in thickness was used as a supporting member.

2. Reagent layer:

Solutions 1, 2, and 3 of the compositions shown below were prepared. A filter paper 260 μm in thickness (produced by Advantec Corp. and marketed under trademark designation of "ADVANTEC No. 2") adopted as a carrier was first impregnated with the solution 1, dried (40°C and 60 minutes), then impregnated with the solution 2, dried (40°C and 10 minutes), and subsequently impregnated with the solution 3, dried (40°C and 10 minutes) to prepare a reagent layer for a testing device for detection of glucose in urine. This reagent layer was cut in the same size as the supporting member mentioned above.

<u>Solution 1</u>

Composed of 3.0 g of glucose oxidase, 100 mg of peroxidase, 150 mg of Tartrazine, 30 ml of aqueous 10% polyvinyl pyrrolidone (K30) solution, 70 ml of aqueous solution of 5% of half ethyl ester of methylvinyl ethermaleic anhydride copolymer (Gantrez AN139), 70 ml of citric buffer solution (pH 5.5), and 100 mg of sodium metaperiodate (oxidizing agent).

<u>Solution 2</u>

Composed of 1.0 g of o-trizine (indicator for coloration by oxidation), 80 mg of 2-mercaptobenzimidazole (stabilizer), and 100 ml of acetone.

<u>Solution 3</u>

Composed of 550 mg of ethyl cellulose and 100 ml of benzene-ethanol mixture (4 : 1).

3. Developing layer:

A polyethylene terephthalate mesh produced by NBC Kogyo K.K. and marketed under product code of "TB 40" was prepared. The diameter of fibers used in this mesh was about 80 μm and the size of openings in the mesh was 50 threads per inch. Then, a developing layer was obtained by immersing the mesh in an aqueous 1% Triton X-100 solution as a surfactant and drying the resultant wet mesh at 40°C for 60 minutes. This developing layer was cut in the same size as the supporting member mentioned above.

4. Sample-absorbing layer:

A sample-absorbing layer formed of woven fabric was packed inside a nipping part of a second retaining piece to be described below. The thickness of the sample-absorbing layer was about 650 μm.

5. Retaining member:

A retaining member composed of a first retaining piece and a second retaining piece of the following conditions was produced.

Material for first and second retaining pieces: Polycarbonate

Size of retaining member: 24 mm (L) x 24 mm (W)

Diameter of opening for sample supply: 6 mm

Diameter of opening for optical measurement: 5 mm

Layout of small holes: 6 small holes disposed round the opening for sample supply

Diameter of small holes: 1 mm

Height of protuberance: 0.8 mm

Control:

A testing device identical to the testing device of this invention described above except for the omission of small holes in the retaining member was produced.

Experiment 1:

The testing devices of the example of this invention and the control described above were used for a sample (blood serum) containing grape sugar to determine the conditions of coloration consequently generated. This experiment was carried out as follows. First, a glucose dope was prepared by dissolving glucose in physiological saline solution in a concentration of 5,000 mg/dl and heat-treating the resultant solution at 37°C for two hours thereby inducing isomerization of the grape sugar. Then, a commercially available standard blood serum (produced by Biorad Corp and marketed under trademark designation of "Life-o-Check Biochemical Control Blood Serum I") was tested for glucose concentration by the use of a commercially available testing kit (produced by Wako Junyaku K.K. and marketed under trademark designation of "Glucose Test Wako"). On the basis of the result of this test, the grape sugar dope was added to the standard blood serum in amounts calculated to produce three blood serum samples, No. 1, 2, and 3, having grape sugar concentrations respectively of 100, 250, and 400 mg/dl.

The testing devices of the example and the control were produced 10 pieces each. The three blood serum samples, No. 1 to 3, were added dropwise in a fixed size of 10 μl to the openings of the test devices for sample supply, left standing in the test devices for one minute. With the aid of an analyzer (produced by Nippon Bunkosha K.K. and marketed under trademark designation of "UVIDEC-610"), projection of light and reception of the reflected light were carried out on the supporting member side to determine the intensity of the reflected light of a wavelength of 541 nm and the concentration of glucose.

The results are shown in Table 1 (example of this invention) and Table 2 (control). The numerical values given in the tables represent glucose levels (mg/dl).

Table 1 (Example)

| n | No. 1 | No. 2 | No. 3 |
|---|---|---|---|
| 1 | 98 | 245 | 370 |
| 2 | 98 | 259 | 371 |
| 3 | 98 | 268 | 386 |
| 4 | 93 | 266 | 370 |
| 5 | 88 | 263 | 374 |
| 6 | 96 | 258 | 381 |
| 7 | 100 | 265 | 373 |
| 8 | 90 | 253 | 361 |
| 9 | 92 | 263 | 372 |
| 10 | 96 | 263 | 371 |
| Average value $\overline{x}$ | 94.9 | 260.3 | 372.9 |
| Standard deviation SD | 3.957 | 6.913 | 6.707 |
| Coefficient of Variation CV (%) | 4.17 | 2.66 | 1.40 |

Table 2 (Controls)

| n | No. 1 | No. 2 | No. 3 |
|---|---|---|---|
| 1 | 99 | 265 | 397 |
| 2 | 101 | 271 | 382 |
| 3 | 96 | 264 | 377 |
| 4 | 98 | 276 | 396 |
| 5 | 88 | 282 | 397 |
| 6 | 96 | 265 | 360 |
| 7 | 96 | 271 | 363 |
| 8 | 96 | 272 | 373 |
| 9 | 92 | 256 | 383 |
| 10 | 90 | 255 | 375 |
| Average value $\bar{x}$ | 95.2 | 267.6 | 377.6 |
| Standard deviation SD | 4.050 | 8.475 | 12.59 |
| Coefficient of Variation CV (%) | 4.25 | 3.17 | 3.33 |

It is noted from Table 1 that the data obtained with the testing devices conforming to this invention were dispersed only sparingly and, therefore, represented results of accurate determination. The reason for the accurate determination is that the small holes formed in the retaining member allowed ample and stable supply of oxygen necessary for the reaction of coloration.

In contrast, with the devices of the control, the times for the reaction of coloration were notably long and the data were appreciably dispersed as shown in Table 2 as compared with those obtained with the devices of the control.

Experiment 2:

The blood serum sample, No. 1 was added dropwise in an excess amount of 100 µl to the opening for sample supply in each of the testing devices of this invention and the control. The condition of absorption of the added sample was kept under visual observation. While the testing devices of the control required a long time (about 8 seconds) for the added sample to be wholly absorbed, the testing devices conforming to this invention required a shorter time for the added sample to be wholly absorbed because the sample was absorbed additionally through the small holes.

## Claims

1. A testing device (1), comprising a testing layer (2) provided with a reagent layer (4) capable of reacting with a specific component in a sample and consequently assuming a pertinent color, a retaining member (8) provided with an opening (7b) for supply of said sample and adapted to support said testing layer (2), and small air-passing holes (7c) formed in said retaining member (8) and opening into the ambient air.

2. A testing device according to claim 1, wherein said retaining member (8) is composed of a pair of mutually fitting retaining pieces (6,7) and said testing layer (2) is nipped between said retaining pieces (6,7).

3. A testing device according to claim 1 or 2, wherein said small air-passing holes (7c) are formed as disposed round said opening (7b).

4. A testing device according to claim 2, wherein said opening (27b) is formed in either of said pair of retaining pieces (27) and said small air-passing holes (26c) are formed in the other retaining piece (26).

5. A testing device according to any one of claims 1 to 4 wherein said small air-passing holes (7c) have a diameter in the range of from 0.5 to 5 mm.

6. A testing device according to any one of claims 1 to 5, wherein said testing layer (2) is provided with a supporting member (3) possessing perviousness to light.

7. A testing device according to any one of claims 1 to 6, wherein said opening (7b) has a diameter in the range of from 1 to 20 mm.

8. A testing device according to claim 7, wherein the number of said small air-passing holes (7c) is in the range of from 1 to 100.

9. A testing device according to any one of claims 1 to 8, wherein said testing layer (2) is provided with a developing layer (5) adjoining said reagent layer (4).

10. A testing device according to any one of claims 1 to 9, wherein said reagent layer (4), is produced by impregnating a porous material with a reagent for testing.

11. A testing device according to claim 9, wherein said reagent layer (4) is provided with a sample-absorbing layer adjoining said developing layer(5).

FIG. 1

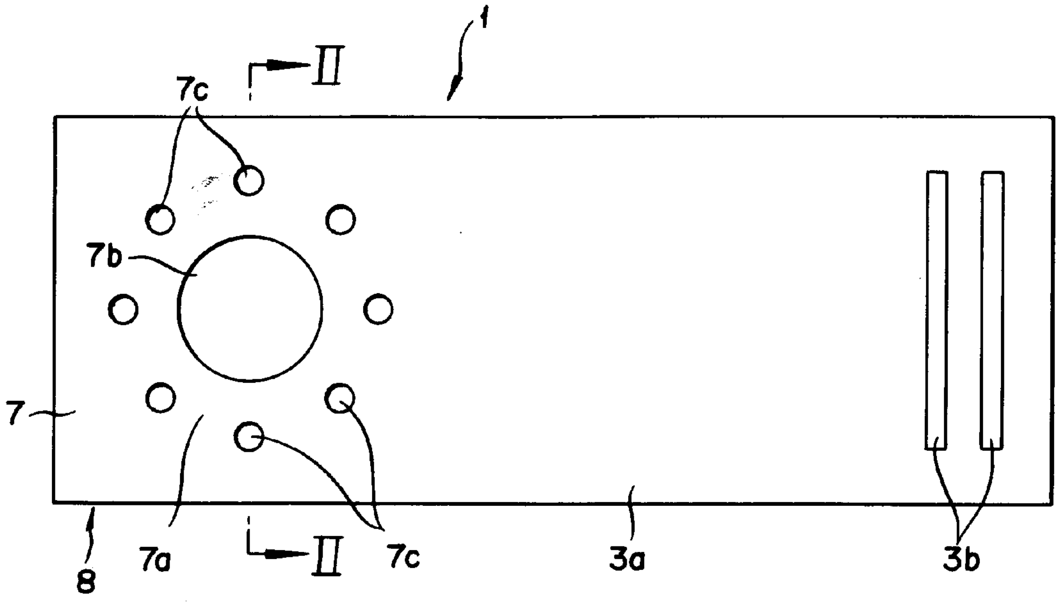


FIG. 2

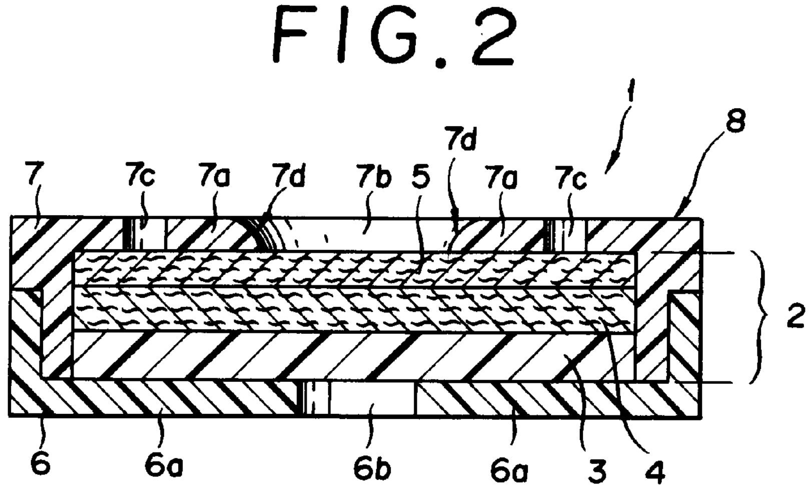

FIG. 3

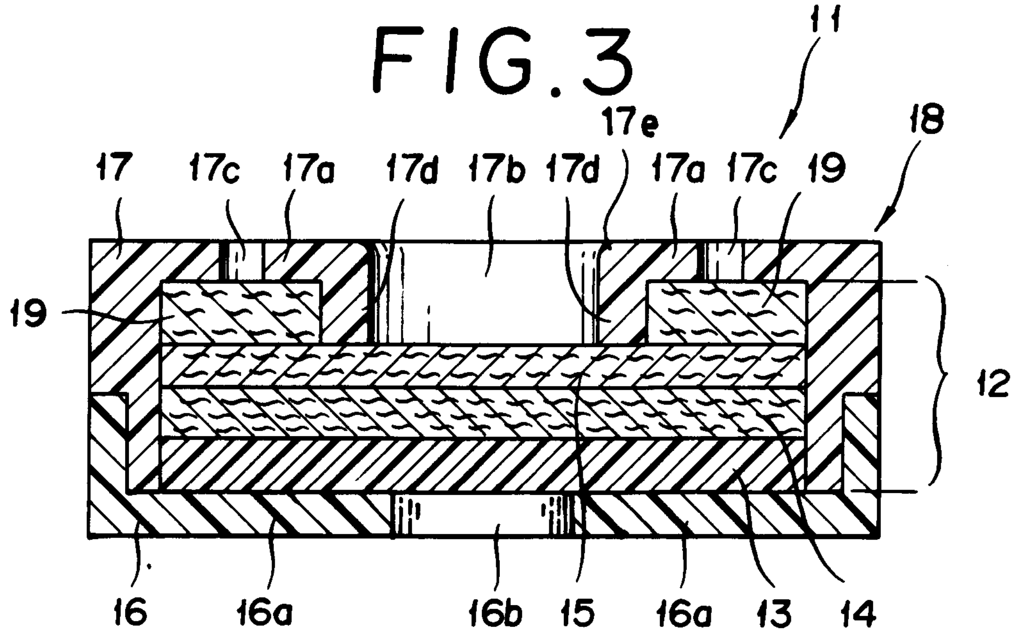
11
17e
18
17
17c
17a
17d
17b
17d
17a
17c
19
19
12
16
16a
16b
15
16a
13
14

FIG. 4

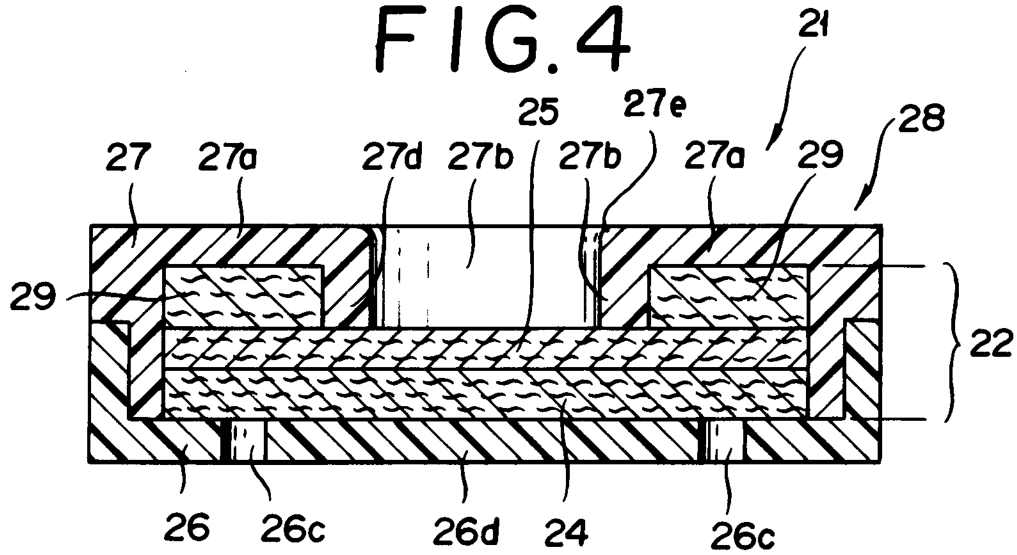
21
25
27e
28
27
27a
27d
27b
27b
27a
29
29
22
26
26c
26d
24
26c

European Patent Office

# EUROPEAN SEARCH REPORT

Application Number

EP 92 40 1196

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 034 049 (EASTMAN KODAK COMPANY)<br>* the whole document *<br>--- | 1-3,7-11 | G01N33/52 |
| X | EP-A-0 283 613 (GENESIS LABS, INC.)<br>* column 1 - column 5 *<br>--- | 1,2,5,8,10 | |
| X | GB-A-2 180 645 (ENVIRONMENTAL DIAGNOSTICS, INC.)<br>* the whole document *<br>--- | 1,2,8,10 | |
| X,P | EP-A-0 451 981 (CASCADE MEDICAL, INC.)<br>* page 1 - page 5 *<br>--- | 1,8 | |
| A | EP-A-0 097 952 (FUJI PHOTO FILM CO., LTD.)<br>* page 23 - page 27; claims; figures *<br>--- | 1-11 | |
| A,P | EP-A-0 435 089 (MILES INC.)<br>--- | | |
| A,D | US-A-4 631 174 (A. KONDO)<br>--- | | |
| E | EP-A-0 487 068 (KYOTO DAIICHI KAGAKU CO., LTD.)<br>* the whole document *<br>----- | 1,2,6,8,10,11 | TECHNICAL FIELDS SEARCHED (Int. Cl.5 )<br>G01N |

The present search report has been drawn up for all claims

| Place of search | Date of completion of the search | Examiner |
|---|---|---|
| THE HAGUE | 29 JULY 1992 | HITCHEN C.E. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)